# EUROPEAN PATENT APPLICATION

(11) **EP 1 894 595 A1**
(43) Date of publication of application: **05.03.2008**
(21) Application number: 07253375.5
(22) Date of filing: 28.08.2007
(51) Int. Cl.: A61M 25/00, A61M 25/01, A61M 25/10

(54) **Catheter tip configuration for improved crossability and trackability**

(30) Priority: 29.08.2006 US 468200
(71) Applicant: Cordis Development Corporation, Miami Lakes, FL 33014 (US)
(72) Inventor: Majercak, David C., Stewartsville, NJ 08886 (US); Hojeibane, Hikmat, Princeton, NH 08540 (US)
(74) Representative: Tunstall, Christopher Stephen

(57) **Abstract**

Balloon catheter designs are disclosed where the as-molded distal tip portion assumes a generally non-linear configuration. The non-linear tip configuration minimizes the gap between the wall of the catheter and the guidewire when the catheter passes over the guidewire when threaded through tortuous portions of the vasculature. The edge of the tip portion may be bias-cut angled with respect to the axis of the catheter to further reduce the gap. The disclosure also describes methods to use the catheter designs.

## Description

### Technical Field

The disclosure relates to the design of catheters that pass over guidewires in the vasculature and that improve crossability and trackability during use.

### Background

The intraluminal delivery of diagnostic catheters, treatment fluids, expansion devices or stents is commonly used to diagnose or treat defects, such as blockages and stenoses, within the human vasculature. Expansion devices can take a number of forms, including a balloon that is inflated to open the blockage. The use of a balloon may provide only a temporary solution and a stent may be inserted after or instead of the balloon as a more permanent solution.

The expansion devices are generally delivered to the blockage by a catheter but catheters cannot define their own path through the vasculature because the path is typically tortuous and may additionally pass through other constrictions in the vasculature. Instead, a more rigid guidewire is first passed through the vasculature to the desired site, then the catheter is passed over the guidewire until the treatment site is reached. A stent delivery system or catheter usable in such a procedure is commonly referred to as an "over-the-wire" or OTW catheter.

An alternative catheter or stent delivery system commonly referred to as a "rapid exchange" or RX catheter also uses a guidewire to properly position the distal end of a catheter. Examples of known RX catheters and methods of using the same are illustrated in U.S. Patent No. 5,061,273 to Yock, which is hereby incorporated herein by reference.

When discussing catheter performance, the related parameters trackability and crossability are often assessed. Both parameters are affected by the properties of most distal (tip) portion of the catheter. An optimal design of this portion would allow the catheter to more easily follow the path of the vasculature (trackability) and to more readily traverse narrow constrictions in the vasculature (crossability). When a guidewire is used, the most distal portion of the catheter also must closely follow the path of the guidewire.

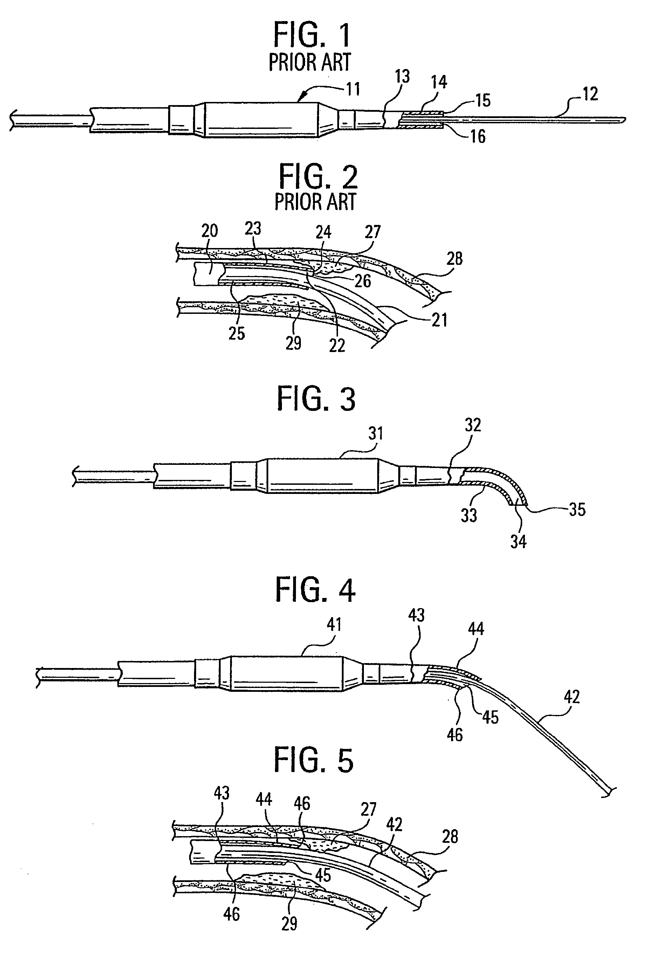

Fig. 1 illustrates a catheter of a type well-known in the art when the catheter is being passed over a guidewire in a relatively straight portion of the vasculature. The distal portion of the catheter 11 has a tip portion 13. The catheter and tip have an lumen 16 through which a guidewire 12 passes. The tip edge 15 is straight cut such that it is perpendicular to the longitudinal axis of the catheter. The tip portion 13 is generally linear and may be made of a flexible material. In this view, the most distal section of the tip is cut away to show the lumen 16 and the wall of the tip 14.

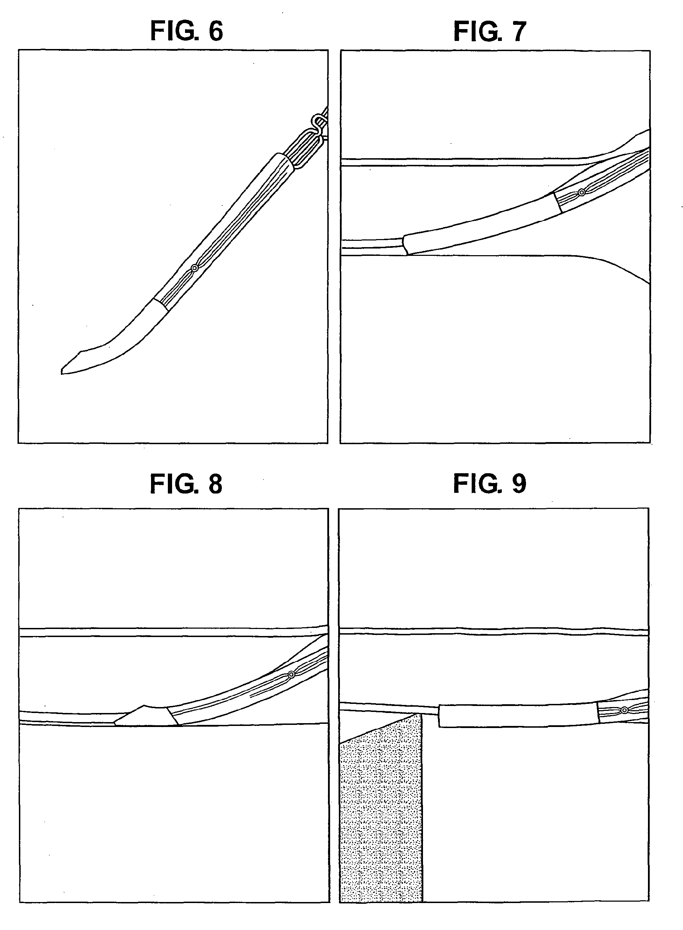

In Fig. 2, a prior art catheter 20 is shown when the catheter tip 20 passes over a guidewire 21 that is curved or bent due to the path that the guidewire 21 takes in the vasculature. A similar view is shown in Fig. 7. The catheter tip wall 23 and tip edge 24 are shown. In this view, the most distal portion of the tip is cut away revealing the lumen 25 and the presence of a gap 26 between the inner face of the wall of the tip 22 and the guidewire 21. The gap 26 can engage with plaque or calcification 27 on an inside curve of a vessel wall 28. Additional plaque 29 is not thus engaged due to its more fortuitous placement this illustration.

We have come to recognize this gap and to appreciate that the gap 26 arises when the catheter tip that had been molded straight bends to follow the path of the guidewire 21 but does not bend to completely the same degree throughout. Crossability and trackability are thereby interfered with due to increased profile at the gap or in its vicinity. Also, the size of the gap may increase with an increase in the degree of bend of the guidewire. The presence of the gap at the leading edge of the catheter typically hinders the passage of a catheter such as a balloon catheter through very narrow sites in the vasculature or at sites where there may be blockages. Furthermore, tissue or other biological material may accumulate in the gap, affecting performance of the catheter.

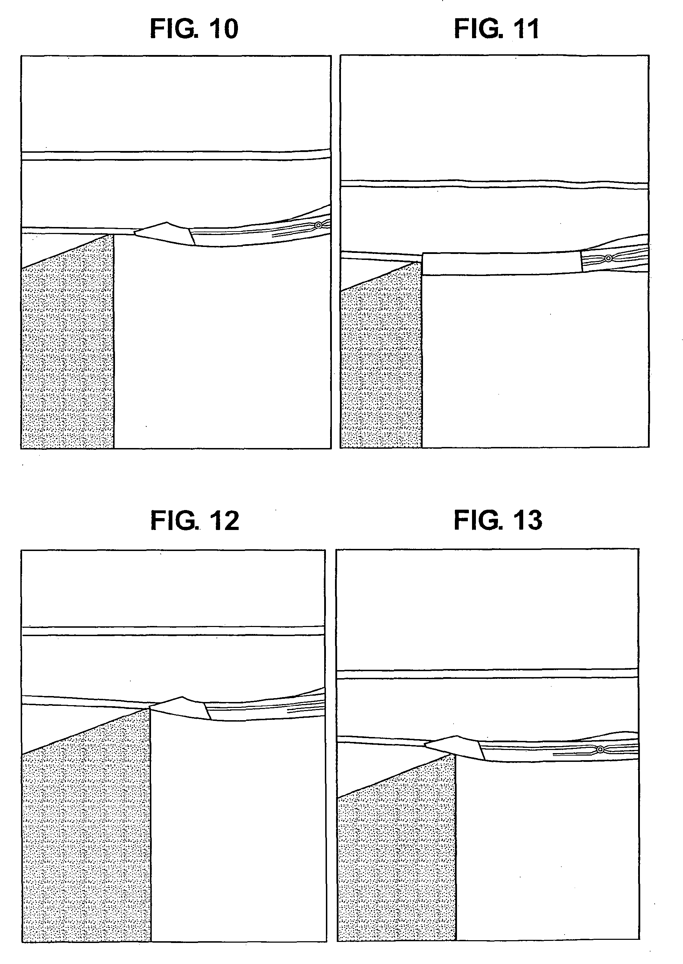

This traditional catheter tip structure also hinders passage at internally protruding calcifications along a curved vessel. Forward progression of the tip is hindered within hard to traverse curved vessels where the tip gets caught on internal calcifications. Fig. 9 shows a traditional catheter tip approaching a mock-up of a calcification protrusion within a vessel. Fig. 11 shows subsequent distal movement where the traditional tip is stopped by the protrusion and would require significant force to pass the protrusion.

When a traditional tip such as that illustrated in Figs. 1, 2, 7, 9 and 11 is "snagged" during insertion into the vasculature of a patient, at least two negative situations may arise. Lack of pushability will prevent or significantly impede the tip and thus the catheter from surpassing the blockage. Also, the tip itself can be damage while passing over the blockage, which then has the potential of causing vessel trauma.

There remains a need for a catheter design that enhances crossablity and trackability through tortuous paths and over calcifications and other protrusions. We have recognized that the presence of this gap condition between the edge of a catheter and the guidewire over which it is passing negatively impacts these key operability parameters, and a problem remains as to how to address the reduced performance issues that arise due to the gap.

### SUMMARY

The present disclosure is directed to medical catheter designs that minimize the gap between the tip of the catheter and the guidewire when the catheter is being advanced through the vasculature. An elongated catheter tubular shaft has a proximal length and a distal end portion with a tip portion at the distal end of the catheter. The tip portion has proximal and distal ends and is generally non-linear in a longitudinal direction when the catheter is not present in the vasculature. In an embodiment, the catheter is provided with the tip portion being generally non-linear in a longitudinal direction, and the distal tip edge thereof is at an orientation that is not perpendicular to the tip portion but is angled with respect to the catheter distal tip.

The disclosure is also directed to methods for using a catheter that has a gap-minimizing configuration. According to one embodiment, the distal tip portion of a catheter with proximal and distal ends and a lumen adapted to pass over a medical device guidewire is provided with an improved distal tip portion. The distal tip portion has proximal and distal ends and has a non-linear configuration in a generally longitudinal direction. The catheter is advanced over a guidewire in the vasculature of a patient, and when the guidewire changes direction the catheter is orientated such that the non-linear tip portion is in the same direction as the guidewire.

### BRIEF DESCRIPTION OF THE DRAWINGS

Fig. 1 is a side view of a prior art balloon catheter and guidewire;

Fig. 2 is a side view of a prior art balloon catheter and guidewire passing through a vascular curve, illustrating the presence of a gap when the catheter passes over the guidewire in a tortuous section of the vasculature;

Fig. 3 is a side view of an embodiment of a balloon catheter according to the present disclosure when not passing over a guidewire;

Fig. 4 is a side view of an embodiment of a balloon catheter according to one embodiment of the present disclosure;

Fig. 5 is an enlarged side view of an embodiment of a balloon catheter according to the present disclosure passing through a vascular curve;

Fig. 6 is a photograph of a catheter tip portion incorporating an embodiment according to the present disclosure;

Fig. 7 is a photograph of a tip portion of a prior art catheter navigating an angled path over a guidewire;

Fig. 8 is a photograph of the catheter portion of Fig. 6 navigating an angled path over a guidewire;

Fig. 9 is a photograph of the catheter portion of Fig. 7 approaching a calcification-like protrusion;

Fig. 10 is a photograph of the catheter portion of Fig. 6 approaching the protrusion as in Fig. 9;

Fig. 11 is a photograph of the catheter portion of Fig. 7 pushed up against the calcification-like protrusion and stopped thereat and not able to pass without significant force;

Fig. 12 is a photograph of the catheter portion of Fig. 6 showing its angle tip contacting the calcification-like protrusion; and

Fig. 13 is a photograph of the catheter portion of Fig. 6 following movement beyond that shown in Fig. 12 with the angle tip easily passing over the calcification-like protrusion.

### DESCRIPTION OF THE EMBODIMENTS

As required, detailed embodiments are disclosed herein; however, it is to be understood that the disclosed embodiments are merely exemplary. Therefore, specific details disclosed herein are not to be interpreted as limiting, but merely as a basis for the claims and as a representative basis for teaching one skilled in the art to variously employ the embodiments disclosed here in virtually any appropriate manner.

Fig. 3 shows one embodiment of a catheter 31 according to the present disclosure. While the figures illustrate balloon catheters, other catheter systems are contemplated, including stent delivery systems. In this view of Fig. 3, the catheter 31 is shown as it would be without the presence of a guidewire. That is, the catheter is shown as it would be when the catheter tip 32 is not stressed, typically when in its as-molded configuration. The catheter tip 32 is orientated so that its axis is curved radially. The portion of the lumen 34 found in the tip follows this radially curved axis while its proximal end remains aligned with the lumen of the catheter. This can be characterized as a bent tip. The illustrated catheter tip 32 is formed on the bias. The catheter end tip edge 35 is angled in that it is not perpendicular relative to the longitudinal axis of the catheter lumen proximal to the tip 32. Fig. 6 also shows these features.

Fig. 4 is an embodiment of a catheter 41 of the present disclosure where the catheter tip 43 with a bent configuration and bias-formed edge has a wall 44 and is shown passing over a guidewire 42 as would occur in the vasculature. Fig. 8 provides a similar illustration. In Fig. 4 view the most distal portion of the tip 43 is cut away to show the lumen 45. With the guidewire within the tip, the bias-formed tip edge 46 is not strictly perpendicular to, but is angled with respect to, the longitudinal axis of the lumen 45 closely proximal of the tip. This is a typical in-use relationship between guidewire and bent tip and gives advantageous gap-minimizing effects as discussed herein.

According to this illustration or embodiment, the non-linear catheter tip 43 is orientated in the direction that the guidewire takes in the vasculature, including over tortuous paths. Due to the bent tip 43 and the bias-formed angled tip edge 46, there is a substantial reduction in the gap between the inner face of the wall of the tip 43 and the guidewire 42 when compared with the gap 26 of the catheter tip shown in Fig. 2 with its tip portion that is neither curved nor with a bias-formed edge. As shown in Fig. 4, this gap reduction or substantial elimination occurs even at a location where the curve of the guidewire and the as-molded curve of the tip portion 43 do not precisely coincide. In actual use, there often is not precise coincidence between catheter tip curve and guidewire curve at any given guidewire length.

Fig. 5 is an enlarged view of the most distal portion of the embodiment in Fig. 4 showing the catheter tip 43 with a bias-formed angled catheter tip edge 46, the most distal portion being cut away to show the wall 44 of the catheter and the lumen 45. The tip wall 44 in this illustration engages the guidewire 42, and the tip edge 46 is closely spaced from the guidewire while substantially following its curved profile within the vasculature. This shows the substantial reduction in the "gap" 46 between the inner face of the wall 44 of the tip 43 and the guidewire 42. To the extent any gap 46 is formed, it is directed away from plaque or calcification 27, thus avoiding aggressive engagement of the plaque 27 or damage to the catheter tip as discussed herein.

It will be appreciated that the bias-formed end has the characteristic of having its inside curved wall length be less than its outside curved wall length. Also, the curvature of this distal tip portion often will be greater than that of the guidewire at a tortuous curve. As a result, the portion of the tip that is less likely to directly engage the guidewire in the typical in-use situation illustrated in Fig. 4 is substantially shorter than the portion most likely to engage the guidewire when in use. Because of this relationship, the location most susceptible to gap formation has the least area and wall length that would otherwise extend away from the guidewire and create an undesireable gap, as illustrated in broken lines in Fig. 5.

According to different embodiments, the tip may be radially bent to different degrees. The degree of bend or curve may be from about 10 to about 90 degrees from the longitudinal axis of the body lumen, or from about 25 to 75 degrees from the longitudinal axis of the body lumen, often between about 35 to 60 degrees from the longitudinal axis.

Typically, gap minimization and crossability and trackability enhancement are facilitated when this catheter tip curve angle is greater than a guidewire curve expected to be encountered during a particular medical procedure within a particular patient. This helps to ensure that the outside (or longer) wall curved length engages the guidewire during use while the portion least likely to engage the guidewire is shortest, so as to minimize the likelihood of unacceptable gap development.

According to different embodiments, the tip edge may be angled or bias-formed to different degrees. The degree of bias-formed angle of the tip edge may be from about 120 to about 240 degrees relative to the longitudinal axis of the body lumen, or from about 140 to about 220 degrees, often between about 160 to 200 degrees from the longitudinal axis of the body lumen. In a preferred embodiment, the tip edge is bias-formed to be 180 degrees relative to the longitudinal axis of the lumen.

Fig. 10 shows the angle tip of the embodiment of this catheter approaching a protrusion mock-up in the form of an intersection point shown closely distal of the angle tip. Fig. 12 illustrates subsequent distal movement and deflecting of the tip upon engagement of the protrusion, which simulates action of the inventive tip when engaging a calcification or other obstruction within a vessel. Fig. 13 shows further distal movement, with the tip easily passing over the protrusion.

The tip portion may be composed of any of a large number of materials known in the art. Ideally, the selected material should allow the tip to bend with the path of the guidewire through the vasculature but also allow the tip to return to its unstressed position when not in use. The tip may be composed, for example, of a polymer material that has adequate bendability or flexural modulus and resiliency.

Shape memory materials may also be used but are not necessary to achieve the benefits of the disclosure. These include so-called nitinol alloys. The bent or curved configuration can be heat set using interlocking dovetails to facilitate flexing. This is illustrated in U.S. Patent Application Publication No. 2004/0082881, hereby incorporated by reference hereinto.

The distal tip portion of the present disclosure may be adapted to fit catheters of different designs. The tip may be used with either over-the-wire or rapid exchange catheters. It may also be used in balloon catheters adapted for stent delivery and expansion.

To use the catheters having the distal tip portions described in the disclosure, the user passes the catheter along a guidewire. When the catheter reaches a region where the guidewire changes direction as it passes through a portion in the vasculature such as a bend, a branched region, or a constriction, the tip will rotate in order to align itself, following what may be said to be a law of least resistance. If constrained for some reason, the tip hits a blockage on the other side, it will pass easily since the tip will hit on the bevel. In essence, the tip configuration will work from both sides, and the tip angle does not need to be oriented in order to align the tip angle with the curve. As a consequence, the distal tip of the catheter more closely follows the guidewire. This brings an advantage of minimizing the gap between the guidewire and the tip.

It will be understood that the embodiments of the present disclosure which have been described are illustrative of some of the applications of the principles of the present disclosure. Numerous modifications may be made by those skilled in the art without departing from the true spirit and scope of the disclosure. Various features which are described herein can be used in any combination and are not limited to procure combinations that are specifically outlined herein.

## Claims

1. A medical catheter having a gap-minimizing configuration, comprising:
an elongated catheter body having a proximal portion, a distal portion and a lumen having a generally longitudinal axis; and
a distal tip portion of the distal body portion, said distal tip portion having:
a proximal portion at said distal portion of said catheter body;
a distal end portion with an opening, said opening terminating with a tip edge;
a distal tip lumen generally aligned with the lumen of the catheter body, whereby said catheter body lumen and said distal tip lumen provide a continuous pathway adapted to pass over a medical device guidewire; and
said distal tip portion has an as-molded configuration that is non-linear with respect to a generally longitudinal direction of the distal body portion.

2. The medical catheter of claim 1, wherein said distal tip portion is curved radially relative to the longitudinal axis of said catheter lumen.

3. The medical catheter of claim 1, wherein said distal tip portion is curved radially from about 10 to about 90 degrees relative to the longitudinal axis of said catheter lumen.

4. The medical catheter of claim 1, wherein said distal end tip edge is formed at a bias so as to be substantially parallel to the longitudinal axis of said catheter lumen.

5. The medical catheter of claim 1, wherein said distal tip portion has an outside curved wall and an inside curved wall, said inside curved wall having a length less than that of said outside curved wall.

6. The medical catheter of claim 1, wherein said distal end tip edge is bias-formed so as to be angled relative to the longitudinal axis of the lumen of said catheter body from about 120 degrees to about 240 degrees.

7. The medical catheter of claim 1, wherein said distal end tip edge is bias-formed so as to be angled relative to the longitudinal axis of the lumen of said catheter body from about 140 to about 220 degrees.

8. A medical catheter having a gap-minimizing configuration, comprising:
an elongated catheter body having a proximal portion, a distal portion and a lumen having a generally longitudinal axis; and
a tip portion of the distal body portion, said distal tip portion having:
a proximal portion at said distal portion of said catheter body;
a distal end portion with an opening, said opening terminating with a tip edge;
a distal tip lumen generally aligned with the lumen of the catheter body, whereby said catheter body lumen and said distal tip lumen provide a continuous pathway adopted to pass over a medical device guidewire;
said distal tip portion has an as-molded configuration that is non-linear in a generally longitudinal direction; and
said distal end tip edge provides a biased edge and has an outside curved wall and an inside curved wall, said inside curved wall having a length less than that of said outside curved wall.

9. The medical catheter of claim 8, wherein said distal tip portion is bent radially relative to the longitudinal axis of said catheter lumen.

10. The medical catheter of claim 8, wherein said distal tip portion is bent radially from about 10 to about 90 degrees relative to the longitudinal axis of said catheter lumen.

11. The medical catheter of claim 8, wherein said distal end tip edge is angled relative to the longitudinal axis of the lumen of the catheter body from about 120 degrees to about 240 degrees.

12. The medical catheter of claim 8, wherein said distal end tip edge is angled at relative to the longitudinal axis of the lumen of the catheter body from about 160 to about 200 degrees.

13. The medical catheter of claim 8, wherein said distal tip portion is made of shape memory material that is heat set to provide the as-molded non-linear configuration of the distal tip portion.
